# EUROPEAN PATENT SPECIFICATION

(11) **EP 3 700 596 B1**
(45) Date of publication and mention of the grant of the patent: **04.06.2025**
(21) Application number: 18797264.1
(22) Date of filing: 10.10.2018
(51) Int. Cl.: A61M 1/00, A61F 13/05, A61F 13/00

(54) **WOUND DRESSING FOR USE WITH ANTI-BACTERIAL MATERIAL**
WUNDVERBAND ZUR VERWENDUNG MIT ANTIBAKTERIELLEM MATERIAL
PANSEMENT POUR PLAIE À UTILISER DANS UN TRAITEMENT ANTIBACTÉRIEN

(30) Priority: 23.10.2017 US 201762575963 P
(43) Date of publication of application: 02.09.2020
(73) Proprietor: Solventum Intellectual Properties Company, Maplewood, MN 55144 (US)
(72) Inventor: EDWARDS, Thomas, Southampton SO15 5FP (GB); LOCKE, Christopher Brian, Bournemouth BH9 3SD (GB); LONG, Justin Alexander, Bournemouth BH1 1PP (GB)
(74) Representative: Simmons & Simmons
(86) International application number: PCT/US2018/055131
(87) International publication number: WO 2019/083726

(56) References cited:
- WO-A1-2016/126444
- WO-A2-2011/087871
- US-A1- 2010 160 853
- US-A1- 2014 039 425
- US-A1- 2015 245 950
- US-A1- 2016 262 942

## Description

### TECHNICAL FIELD

The invention set forth in the appended claims relates generally to tissue treatment systems and more particularly, but without limitation, to tissue dressings that may be suitable for use with negative-pressure therapy.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times. Improvements to therapy systems, components, and processes may benefit healthcare providers and patients.
WO2011/087871 discloses a reduced pressure connector including a first channel to couple a reduced pressure source to a wound and a second channel to couple an air vent to the wound. US2016/0262942 and US2010/0160853 disclose wound dressings which may include dialkyl carbomoyl chloride.
WO2016/126444 discloses a reduced pressure treatment dressing having a plurality of elongate members.
US2015/0245950 discloses an absorbent, negative pressure, wound treatment system.
Further relevant prior art can be found in US 2014/039425 A1.

### BRIEF SUMMARY

According to claim 1, there is provided an apparatus for treating a tissue site, comprising: a dressing; and a bridge adapted to be fluidly coupled to the dressing, the bridge comprising: a first manifold layer, a second manifold layer, a binding layer comprising a binding material positioned between the first manifold layer and the second manifold layer, wherein the binding material has bacterial-binding and protein-binding properties, and a sealing material having a first end and a second end, wherein the first end comprises an first aperture adapted to be fluidly coupled to a negative-pressure source, and the second end comprises an second aperture adapted to be fluidly coupled to the dressing; wherein the first manifold layer, the second manifold layer, and the binding layer are enclosed within the sealing material and are fluidly coupled between the first and second apertures.

A selection of optional features is set out in the dependent claims.

### BRIEF DESCRIPTION OF THE DRAWINGS

Figure 1 is a functional block diagram of an example embodiment of a therapy system that can provide negative-pressure therapy to a tissue site in accordance with this specification;
Figure 2 is a plan view of an illustrative embodiment of a portion of the system of Figure 1, including a dressing and bridge;
Figure 3A is an exploded view of the bridge of Figure 2, according to some illustrative embodiments;
Figure 3B is an exploded view of the bridge of Figure 2, according to some additional illustrative embodiments;
Figure 4 is a cross-section of a portion of an illustrative embodiment of the bridge of Figure 2;
Figure 5 is an exploded view of a dressing, according to some illustrative embodiments, suitable for use with the system of Figure 1, depicted without a conduit interface and with an illustrative embodiment of a release liner for protecting the dressing prior to application at a tissue site;
Figure 6 is a line graph illustrating the results of an experiment comparing measured pressure differentials of at least one exemplary embodiment of a bridge and a control dressing structure;
Figure 7 is a line graph illustrating the results of an additional experiment comparing measured pressure differentials of at least one exemplary embodiment of a bridge and a control dressing structure; and
Figure 8 is a line graph illustrating the results of an additional experiment comparing pressure differentials of exemplary embodiments of a bridge.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

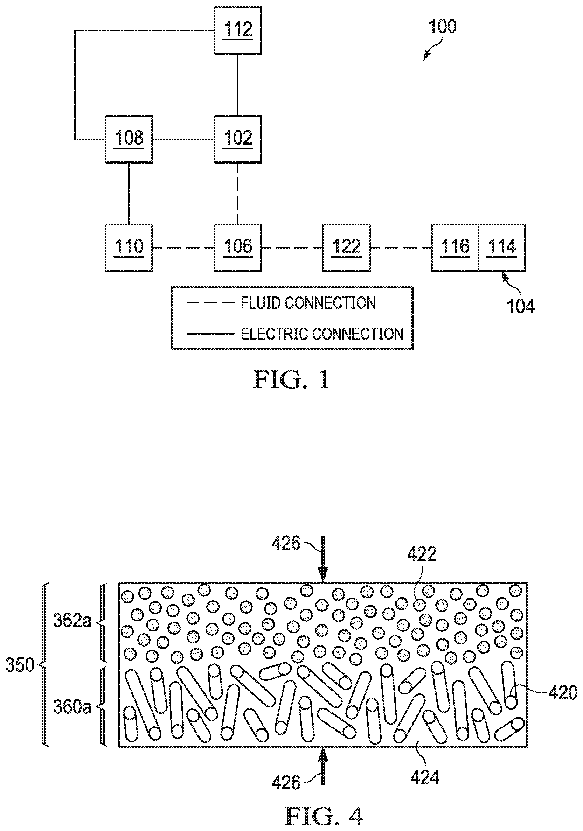

Figure 1 is a simplified functional block diagram of an example embodiment of a therapy system 100 that can provide negative-pressure therapy to a tissue site in accordance with this specification.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 102, a dressing 104, a fluid container, such as a container 106, and a regulator or controller, such as a controller 108, for example. Additionally, the therapy system 100 may include sensors to measure operating parameters and provide feedback signals to the controller 108 indicative of the operating parameters. As illustrated in Figure 1, for example, the therapy system 100 may include a pressure sensor 110, an electric sensor 112, or both, coupled to the controller 108. As illustrated in the example of Figure 1, the dressing 104 may comprise or consist essentially of a tissue interface 114, a cover 116, or both in some embodiments. Also illustrated in the example of Figure 1, the therapy system 100 may include a bridge 122, which may be positioned in fluid communication between the negative-pressure source 102 and the dressing 104.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 102 may be combined with the controller 108 and other components into a therapy unit.

In general, components of the therapy system 100 may be coupled directly or indirectly. For example, the negative-pressure source 102 may be directly coupled to the container 106, and may be indirectly coupled to the dressing 104 through the container 106. Coupling may include fluid, mechanical, thermal, electrical, or chemical coupling (such as a chemical bond), or some combination of coupling in some contexts. For example, the negative-pressure source 102 may be electrically coupled to the controller 108, and may be fluidly coupled to one or more distribution components to provide a fluid path to a tissue site. In some embodiments, components may also be coupled by virtue of physical proximity, being integral to a single structure, or being formed from the same piece of material.

A distribution component is preferably detachable, and may be disposable, reusable, or recyclable. The dressing 104 and the container 106 are illustrative of distribution components. A fluid conductor is another illustrative example of a distribution component. A "fluid conductor," in this context, broadly includes a tube, pipe, hose, conduit, or other structure with one or more lumina adapted to convey a fluid between two ends. Typically, a tube is an elongated, cylindrical structure with some flexibility, but the geometry and rigidity may vary. Moreover, some fluid conductors may be molded into or otherwise integrally combined with other components. Additionally, the bridge 122 is also illustrative of a distribution component, and may be more specifically considered as a fluid conductor. Distribution components may also include or comprise interfaces or fluid ports to facilitate coupling and de-coupling other components. In some embodiments, for example, a dressing interface may facilitate coupling a fluid conductor to the dressing 104. For example, such a dressing interface may be a SENSAT.R.A.C.^{™} Pad, commercially available from KCI, of San Antonio, Texas.

A negative-pressure supply, such as the negative-pressure source 102, may be a reservoir of air at a negative pressure, or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The container 106 is representative of a container, canister, pouch, or other storage component, which can be used to manage exudates and other fluid withdrawn from a tissue site. In many environments, a rigid container may be preferred or required for collecting, storing, and disposing of fluid. In other environments, fluid may be properly disposed of without rigid container storage, and a re-usable container could reduce waste and costs associated with negative-pressure therapy. In some embodiments, exudates and other fluid withdrawn from a tissue site may be managed or stored by the dressing 104 in addition to or instead of the container 106. Thus, in some embodiments, a separate container 106 may be omitted from the therapy system 100 depending on the particular dressing 104 incorporated into the therapy system 100.

A controller, such as the controller 108, may be a microprocessor or computer programmed to operate one or more components of the therapy system 100, such as the negative-pressure source 102. In some embodiments, for example, the controller 108 may be a microcontroller, which generally comprises an integrated circuit containing a processor core and a memory programmed to directly or indirectly control one or more operating parameters of the therapy system 100. Operating parameters may include the power applied to the negative-pressure source 102, the pressure generated by the negative-pressure source 102, or the pressure distributed to the tissue interface 114, for example. The controller 108 is also preferably configured to receive one or more input signals, such as a feedback signal, and programmed to modify one or more operating parameters based on the input signals.

Sensors, such as the pressure sensor 110 or the electric sensor 112, are generally known in the art as any apparatus operable to detect or measure a physical phenomenon or property, and generally provide a signal indicative of the phenomenon or property that is detected or measured. For example, the pressure sensor 110 and the electric sensor 112 may be configured to measure one or more operating parameters of the therapy system 100. In some embodiments, the pressure sensor 110 may be a transducer configured to measure pressure in a pneumatic pathway and convert the measurement to a signal indicative of the pressure measured. In some embodiments, for example, the pressure sensor 110 may be a piezoresistive strain gauge. The electric sensor 112 may optionally measure operating parameters of the negative-pressure source 102, such as the voltage or current, in some embodiments. Preferably, the signals from the pressure sensor 110 and the electric sensor 112 are suitable as an input signal to the controller 108, but some signal conditioning may be appropriate in some embodiments. For example, the signal may need to be filtered or amplified before it can be processed by the controller 108. Typically, the signal is an electrical signal, but may be represented in other forms, such as an optical signal.

The tissue interface 114 can be generally adapted to contact a tissue site. The tissue interface 114 may be partially or fully in contact with a tissue site. If a tissue site is a wound, for example, the tissue interface 114 may partially or completely fill the wound, or may be placed over the wound. The tissue interface 114 may take many forms, and may have many sizes, shapes, or thicknesses depending on a variety of factors, such as the type of treatment being implemented or the nature and size of a tissue site. For example, the size and shape of the tissue interface 114 may be adapted to the contours of deep and irregular shaped tissue sites. Moreover, any or all of the surfaces of the tissue interface 114 may have projections or an uneven, course, or jagged profile that can induce strains and stresses on a tissue site, which can promote granulation at the tissue site.

In some embodiments, the tissue interface 114 may comprise or consist of a manifold. A "manifold" in this context generally includes any substance or structure providing a plurality of pathways adapted to collect or distribute fluid across a tissue site under pressure. For example, a manifold may be adapted to receive negative pressure from a source and distribute negative pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, the pathways of a manifold may be interconnected to improve distribution or collection of fluid across a tissue site. In some illustrative embodiments, a manifold may be a porous foam material having interconnected cells or pores. The average pore size of a foam may vary according to needs of a prescribed therapy. For example, in some embodiments, the tissue interface 114 may be a foam having pore sizes in a range of 400-600 microns. The tissue interface 114 may also wick fluid away from a tissue site, while continuing to distribute negative pressure to the tissue site. The wicking properties of the tissue interface 114 may draw fluid away from a tissue site by capillary flow or other wicking mechanisms. Additionally, in some embodiments, the tissue interface 114 may be constructed from bioresorbable materials.

In some embodiments, the cover 116 may provide a bacterial barrier and protection from physical trauma. The cover 116 may also be constructed from a material that can reduce evaporative losses and provide a fluid seal between two components or two environments, such as between a therapeutic environment and a local external environment. The cover 116 may be, for example, an elastomeric film or membrane that can provide a seal adequate to maintain a negative pressure at a tissue site for a given negative-pressure source. The cover 116 may have a high moisture-vapor transmission rate (MVTR) in some applications. For example, the MVTR may be at least 250 g/m² per twenty-four hours, measured by upright cup technique, according to ASTM E96/E96M Upright Cup Method at 38°C and 10% relative humidity (RH). In some embodiments, the cover 116 may have a MVTR between 250 g/m² per twenty-four hours and 5,000 g/m² per twenty-four hours. In some example embodiments, the cover 116 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained.

The cover 116 may include a sealing material, which may be formed from any material that allows for a fluid seal to be provided. For example, the cover 116 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber;; ethylene vinyl acetate (EVA); co-polyester;, and polyether block polymide copolymers. Such materials are commercially available, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some embodiments, the cover 116 comprises INSPIRE 2301 having an MVTR (upright cup technique) of 2600 g/m²/24 hours and a thickness of about 30 microns

An attachment device may be used to attach the cover 116 to an attachment surface, such as undamaged epidermis, a gasket, or another cover. The attachment device may take many forms. For example, an attachment device may be a medically-acceptable, pressure-sensitive adhesive configured to bond the cover 116 to epidermis around a tissue site. In some embodiments, for example, some or all of the cover 116 may be coated with an adhesive, such as an acrylic adhesive, which may have a coating weight between 25-65 g/m² (gsm). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The fluid mechanics of using a negative-pressure source to reduce pressure in another component or location, such as within a sealed therapeutic environment, can be mathematically complex. However, the basic principles of fluid mechanics applicable to negative-pressure therapy are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

In operation, the tissue interface 114 may be placed within, over, on, or otherwise proximate to a tissue site. The cover 116 may be placed over the tissue interface 114 and sealed to an attachment surface near the tissue site. For example, the cover 116 may be sealed to undamaged epidermis peripheral to a tissue site. Thus, the dressing 104 can provide a sealed therapeutic environment proximate to a tissue site, substantially isolated from the external environment, and the negative-pressure source 102 can reduce the pressure in the sealed therapeutic environment. Negative pressure applied across the tissue site through the tissue interface 114 in the sealed therapeutic environment can induce macrostrain and micro-strain in the tissue site, as well as remove exudates and other fluids from the tissue site, which can be collected in container 106.

An important factor for efficient fluid management across the components of a system for providing negative-pressure therapy, such as therapy system 100, is the proper management of high viscosity fluid. Higher viscosity fluids may be more likely to cause occlusions or become resistant to movement within some wound dressings or other system components. The impairment of fluid movement may cause some dressings or other system components to fill with wound fluid, which may result in creating a pressure differential or drop between the pressure level at a source of negative-pressure and the pressure level at a tissue site. Often, the more difficult types of fluids to manage are the higher viscosity wound fluids, such as fluids having a viscosity of around 30 mPa·s. Such higher viscosity wound fluids often have higher protein content, and following a period of heavy exudate, which may be around 3-4 days into treatment, the proteins may begin to impair the ability of a wound dressing or other components of negative-pressure therapy systems to effectively transport wound fluid and facilitate wound fluid movement out of the wound dressing or other system components. Furthermore, in such dressings or negative-pressure devices or systems that include components designed to at least partially evaporate fluid, the higher protein content may also begin to interfere with the evaporation of wound fluid from a dressing or other components of a therapy system. This reduction in evaporation of fluid may exacerbate pressure drops in one or more dressing or system components.

Negative-pressure dressings or systems which may be particularly susceptible to occlusions and resulting pressure drops may be those which include a long, slender, vertical component, such as a conduit or other fluid conductor, between a source of negative pressure and a base wound dressing, which may be positioned at a relatively lower vertical position on a patient and perhaps underneath a compression garment. For example, dressings and other aspects of negative-pressure systems used for the treatment of a venous leg ulcer (VLU) may be vulnerable to such pressure drops. VLUs often produce protein-rich fluid having a relatively high viscosity. Given that it is often preferable for target wear times of some dressings for VLUs and other types of tissue sites, as well as overlying compression garments, to be up to 7 days before changing, such occlusions and pressure drops prior to the conclusion of the 7-day period may be particularly disruptive to patient lifestyle and healing, as the dressing components may need to be discarded. Costs may also be increased due to the possible discarding of both the dressing components as well as other components of the negative-pressure system.

Distribution components of the therapy system 100 can manage high-viscosity fluid, particularly protein-rich fluid of varying viscosities produced by VLUs. In some embodiments of the therapy system 100, a dressing or conduit structure for mitigating the effects of high protein content in viscous wound exudate may be provided. Such a structure may include a material that can bind particular components associated with wound exudate, including among others, bacteria and one or more types of protein.

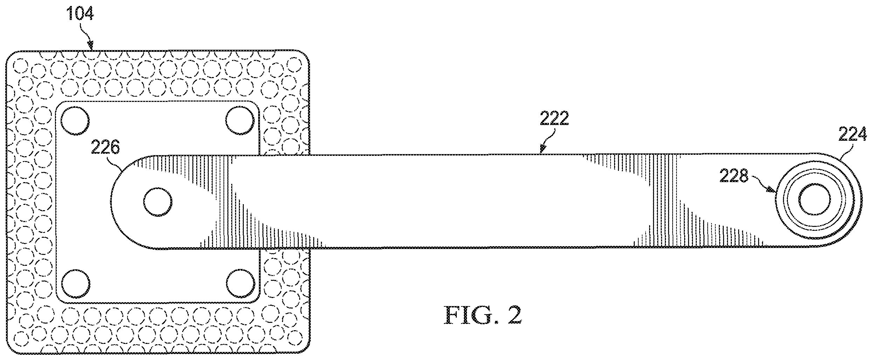

Figure 2 is a schematic diagram illustrating additional details that may be associated with some example embodiments of the therapy system 100. In the example embodiment of Figure 2, a dressing 104 and bridge 222 are shown. The bridge 222 is an example embodiment of the bridge 122 of Figure 1. The bridge 222 may fluidly couple the dressing 104 and the negative-pressure source 102 in any suitable manner. In some embodiments, the bridge 222 may have a first end 224 and a second end 226. The length of the bridge 222 may be any length suitable for a particular application to a tissue site, such as a VLU. For example, in some embodiments, the length of the bridge 222 may be between about 300 millimeters and about 1200 millimeters. In some embodiments, the bridge 222 may also include an attachment port 228 for fluidly connecting a source of negative pressure, such as negative-pressure source 102, to the bridge 222. For example, in some embodiments, the attachment port 228 may be configured to be coupled to a fluid conduit, such as a tube, which may terminate in an adapter for fluid connection to the negative-pressure source 102.

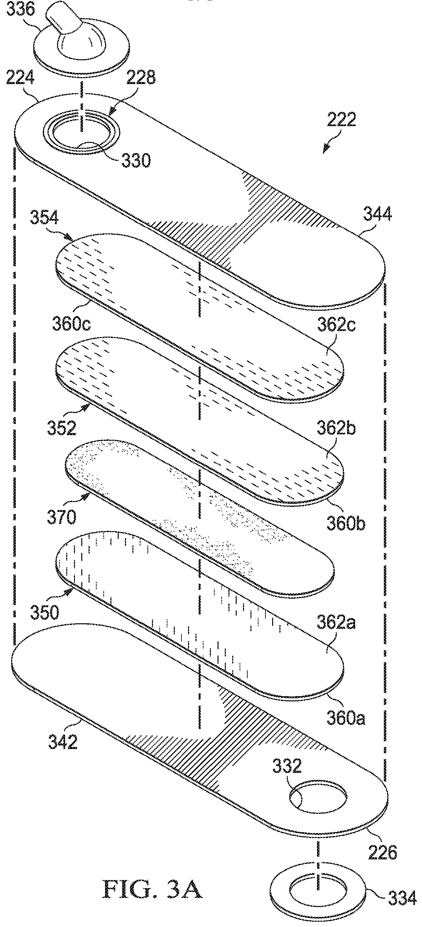

Figure 3A is an exploded view of the bridge 222 of Figure 2, showing additional details and features that may be associated with some illustrative embodiments. The bridge 222 may include a sealing member, which may be formed of one or more layers of sealing material, such as a first sealing layer 342 and a second sealing layer 344. The first sealing layer 342 may have a first periphery bonded to a second periphery of the second sealing layer 344. In some embodiments, the first periphery of the first sealing layer 342 may be welded or joined with an adhesive to the second periphery of the second sealing layer 344. Between the first sealing layer 342 and the second sealing layer 344 may be an internal passageway. Within the internal passageway, the bridge 222 may include one or more layers of a manifold material. For example, the layers of manifold material may be encapsulated or sealingly enclosed between the first sealing layer 342 and the second sealing layer 344 and also between the first end 224 and the second end 226 of the bridge 222. In some embodiments, the first sealing layer 342 may have a first periphery bonded to a second periphery of the second sealing layer 344 around the layers of manifold material in any suitable manner. Additionally or alternatively, the sealing member may be formed of a single layer of sealing material, which may be folded or wrapped around the other components of the bridge 222 and joined and sealed along two edges of the single layer of sealing material. The two edges of the single layer of sealing material may be joined through welding, the use of an adhesive, or other attachment means. Furthermore, the sealing member may also be in the form of a sleeve, which may be produced using an extrusion or other process.

The one or more layers of sealing material, such as the first sealing layer 342 and the second sealing layer 344, may be comprised of similar materials described above for the cover 116. For example, the layers of sealing material may each be an adhesive-coated film, such as an adhesive-coated polyurethane film. In some embodiments, the layers of sealing material may be an INSPIRE 2327 or INSPIRE 2301 drape. The structure of the bridge 222 may, in some embodiments, replace the need for including more traditional conduit structures and materials, such as plastic tube sets, in parts of the therapy system 100. Further, other materials for the one or more layers of sealing material may be used, such as polyurethane film, films with and without adhesive, and other high-MVTR films. High-MVTR films may provide for evaporation of condensate. In some preferred embodiments, the bridge 222 may include one or more thin, flexible non-woven manifold material layers sealed between two layers of sealing material, such as the first sealing layer 342 and the second sealing layer 344, which may be polyurethane layers or other occlusive layers which may be bonded or sealed together.

In some embodiments, the first sealing layer 342 may include an adhesive layer on an external surface. For example, the external surface of the first sealing layer 342 may further include a layer of a double-sided adhesive tape, which may have a release liner protecting the external adhesive surface prior to application to the skin of a patient. The adhesive layer may include any suitable adhesive material, including adhesive acrylates, however those adhesives that provide an adequate tack without causing harm to a patient's skin may be most appropriate.

In some embodiments, patterns or shallow ridges may be embossed into the first sealing layer 342 and/or the second sealing layer 344 to aid pressure transfer and further resist crushing. Further, odor-absorbing additives may be added to the bridge 222 to absorb bad-smelling gases and vapors that may be liberated from the wound or dressing.

As previously mentioned, the bridge 222 may include one or more layers of a manifold material encapsulated or sealingly enclosed within the one or more layers of sealing material. For example, the bridge 222 may include a first manifold layer 350, a second manifold layer 352, and a third manifold layer 354. Each of the one or more layers of manifold material may extend along the length of the bridge 222 and may be disposed within the internal passageway that may be defined by the one or more layers of the sealing member. In some embodiments, the bridge 222 may include additional layers of manifold material, for example, a fourth manifold layer and a fifth manifold layer, depending on the particular application. In additional example embodiments, the bridge 222 may include only one or two layers of manifold material.

In some embodiments, the manifold material may include a wicking material. Furthermore, the one or more layers of manifold material may include a non-woven material, such as, for example, a polyester non-woven. In some embodiments, the one or more layers of manifold material may include Libeltex TDL4 co-polyester, commercially available from Libeltex BVBA, Meulebeke, Belgium. In some embodiments, other non-woven materials may be used for the manifold material, such as Freudenberg M1505, commercially available from Freudenberg Group, Weinheim, Germany; a compressed polyolefin, commercially available from Essentra PLC, Buckinghamshire, United Kingdom; or Libeltex TDL2, commercially available from Libeltex BVBA, Meulebeke, Belgium. In additional embodiments, the manifold material may be an open-celled polyurethane foam or laminations with fiber or foam structures.

A periphery or edge of the first manifold layer 350 may be coupled to a periphery or edge of the second manifold layer 352 in any suitable manner, such as, for example, by a weld. A periphery or edge of the second manifold layer 352 may also be coupled to a periphery or edge of the third manifold layer 354 in any suitable manner, such as, for example, by a weld. The second manifold layer 352 may be positioned between the first manifold layer 350 and the third manifold layer 354. In some embodiments, the one or more manifold layers, such as the first manifold layer 350, second manifold layer 352, and third manifold layer 354, may be positioned and sealed between the first sealing layer 342 and the second sealing layer 344 of the sealing member without any welds between the manifold layers.

In some embodiments, the one or more manifold layers, such as the first manifold layer 350, the second manifold layer 352, and the third manifold layer 354, may each include an acquisition side and a distribution side. For example, the first manifold layer 350 may include an acquisition side 360a and a distribution side 362a, the second manifold layer 352 may include an acquisition side 360b and a distribution side 362b, and the third manifold layer 354 may include an acquisition side 360c and a distribution side 362c. The distribution sides 362a-c may be positioned on opposite sides or surfaces of the first manifold layer 350, the second manifold layer 352, and the third manifold layer 354 from the acquisition sides 360a-c. For example, the acquisition sides 360a-c of each of the first manifold layer 350, the second manifold layer 352, and the third manifold layer 354 may face in a direction of the first sealing layer 342. Further, the distribution sides 362a-c of each of the first manifold layer 350, the second manifold layer 352, and the third manifold layer 354 may face in a direction of the second sealing layer 344. However, each or all of the first manifold layer 350, the second manifold layer 352, and the third manifold layer 354 may be oppositely oriented, so that a variety of embodiments may be achieved, with different combinations of the acquisition sides 360a-c and distribution sides 362a-c of the first manifold layer 350, the second manifold layer 352, and the third manifold layer 354 being oriented towards either the first sealing layer 342 or the second sealing layer 344.

The bridge 222 further includes a binding material having bacterial-binding as well as protein-binding properties. The bridge 222 includes a binding layer 370, which is in the form of a layer of binding material, positioned between the first manifold layer 350 and the second manifold layer 352 of the bridge 222. Alternatively, the binding layer 370 may also be positioned between the second manifold layer 352 and the third manifold layer 354. Additional embodiments of the bridge 222 may include multiple layers of the binding material, for example, with a first layer of binding material, such as binding layer 370, positioned between the first manifold layer 350 and the second manifold layer 352 and a second layer of binding material positioned between the second manifold layer 352 and the third manifold layer 354. Additional or alternative embodiments may include different arrangements of layers of the binding material and manifold layers based on the particular need or application.

In some embodiments, the binding layer 370 may be in the form of a mesh. The binding layer 370 may allow for substantially even distribution of negative pressure within the bridge 222, while reducing or preventing the spread of microorganism growth, particularly bacteria, which if left untreated may spread infection in a tissue site. For example, bacteria and other microorganisms may include gram positive bacteria, such as Staphylococcus aureus, MRSA, or Streptococci; gram negative bacteria such as E. coli or Pseudomonas aeruginosa; fungi, such as Candida albicans; as well as others.

The binding layer 370 may be in the form of a sheet or layer, which may include fibers coated with a material having a high affinity for binding bacteria and also proteins. In some embodiments, the binding layer 370 may be in the form of a low-profile layer, or 2-D lattice work coated with binding material, so as to add minimal volume to the bridge 222. For example, the coating material may include dialkyl carbamoyl chloride (DACC) hydrophobic coating, such as used in Cutimed^{®} Sorbact^{®}, commercially available from BSN Medical. The following is a representative chemical structure of DACC. 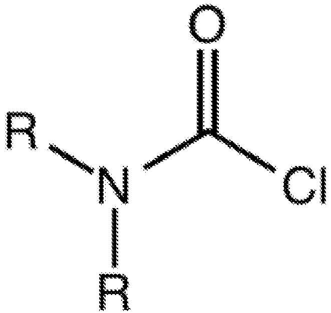 wherein R is lower alkyl, such as methyl.

As is evident from the above chemical structure, DACC includes both nonpolar groups (the methyl (or alkyl) entities), and polar groups (oxygen, nitrogen, and chlorine). Accordingly, the DACC may demonstrate a dipolar character, which is also a characteristic of many proteins. The polar groups of DACC may increase the hydrophilicity of the molecule, which in some instances may be counteracted by increasing the chain length of the alkyl groups. Additionally, molecules with similar polar groups as to those of the DACC molecule could be selected to replicate the dipolar nature.

The binding material of the binding layer 370 may attract the bacteria and proteins through hydrophobic interaction, which is the principle that when two hydrophobic particles come together, they bind with the force of the surrounding water molecules. For example, protein molecules expressing surface hydrophobicity may be attracted to and held by the binding material, thus helping to separate the protein molecules from surrounding water molecules present in the wound exudates. As a result, the protein molecules may remain bound to the binding material and be kept away from blocking the fluid manifolding pathways in the bridge 222 that manifold pressure, move fluids, and ultimately facilitate the evaporation of much of the water present in the wound exudates before it travels the container 106.

Since most bacterial pathogens are hydrophobic, in the semi-aqueous environment of a tissue site, which includes a wound or wound exudates leaving a wound, the binding material and the bacterial pathogens may have an affinity for binding together if the binding material is hydrophobic. If the bacteria are bound to the binding material, the bacteria may be rendered unable to reproduce or release harmful toxins to the tissue site. Furthermore, by including the binding material in a binding layer 370 within the bridge 222, the bacteria may be kept remote from a tissue site; however, the binding material may still be in close enough fluid communication with fluid leaving the tissue site to be effective.

Regardless of the exact positioning of the binding layer 370, the binding layer 370 may offer the greatest benefit if it is between layers of the manifold material, such as for example, between the first manifold layer 350 and the second manifold layer 352 of the bridge 222. In such a configuration, bacteria-rich and/or protein-rich fluid from a tissue site may move along the surface area on both sides of the binding layer 370. Further, the movement of fluid along the sides of the binding layer 370 may be assisted by the wicking or manifolding functionality of the layers of the manifold material. As shown in the illustrative embodiment depicted in Figure 3A, the binding layer 370 may comprise a DACC-coated material and may be disposed between two of the three layers of the manifold material, all of which may be encapsulated within the sealing member.

Still referring primarily to Figure 3A, the bridge 222 may include one or more apertures for being fluidly connected to other components of the therapy system 100. For example, the second sealing layer 344 may include a first aperture 330 at the first end 224 of the bridge 222. Additionally, the first sealing layer 342 may include a second aperture 332 at the second end 226 of the bridge 222. The first end 224 and the first aperture 330 may be in fluid communication with the second end 226 and the second aperture 332 through the length of the bridge 222. In some embodiments, a seal 334 may be positioned about the second aperture 332 and between the second end 226 of the bridge 222 and the dressing 104 for bonding the second end 226 of the bridge 222 to the dressing 104 and for maintaining fluid communication between the bridge 222 and the dressing 104 through the second aperture 332. In some embodiments, a conduit interface 336 may be included and positioned proximate to the bridge 222 and in fluid communication with the attachment port 228 and the first aperture 330 of the first end 224 of the bridge 222. The conduit interface 336 may communicate negative pressure from the negative-pressure source 102 to the bridge 222. The conduit interface 336 may comprise a medical-grade, soft polymer or other pliable material. As non-limiting examples, the conduit interface 336 may be formed from polyurethane, polyethylene, polyvinyl chloride (PVC), fluorosilicone, or ethylene-propylene. In some illustrative, non-limiting embodiments, the conduit interface 336 may be molded from PVC which is free from di(2-ethylhexyl)phthalate (DEHP). The conduit interface 336 may be formed in any suitable manner, such as by molding, casting, machining, or extruding. Further, the conduit interface 336 may be formed as an integral unit or as individual components and may be coupled to the bridge 222 by, for example, adhesive or welding.

In some embodiments, the conduit interface 336 may include an odor filter or material adapted to substantially preclude the passage of odors from the bridge 222. In some embodiments, the odor filter may be comprised of a carbon material in the form of a layer or particulate. For example, the odor filter may comprise a woven carbon cloth filter such as those manufactured by Chemviron Carbon, Ltd. of Lancashire, United Kingdom. Further, in some instances, the conduit interface 336 may carry a hydrophobic filter adapted to substantially preclude the passage of liquids out of the bridge 222. The hydrophobic filter may be comprised of a material that is substantially liquid impermeable and vapor permeable. For example, the hydrophobic filter may comprise a GORE^{™} Medical Membrane MMT-314, commercially available from W.L. Gore & Associates, Inc. of Newark, Delaware. The hydrophobic filter may be in the form of a membrane or layer.

The odor filter and/or hydrophobic filter may be disposed in the conduit interface 336 or other suitable location such that fluid communication between the negative-pressure source 102 and the dressing 104 is provided through the odor filter and/or hydrophobic filter. In some embodiments, the odor filter and/or the hydrophobic filter may be secured within the conduit interface 336 in any suitable manner, such as by adhesive or welding.

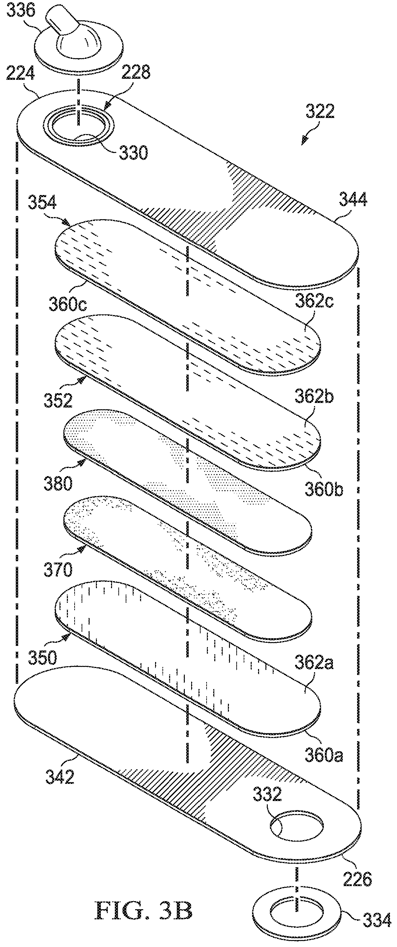

Figure 3B illustrates an additional embodiment of a bridge 322, which may be substantially similar to the bridge 222 shown in Figure 3A, and may be an example embodiment of the bridge 122 of Figure 1. In addition to the components described with respect to the bridge 222 of Figure 3A, the bridge 322 may also include absorbent layer 380. Absorbent layer 380 may include an absorbent component, and in some embodiments, may include a super-absorbent material. The absorbent layer 380 may be positioned next to or sandwiched between the one or more layers of manifold material, for example between the first manifold layer 350 and the second manifold layer 352. In some embodiments, the absorbent layer 380 may also be positioned adjacent to the binding layer 370. Additionally or alternatively, the absorbent layer 380 may also be positioned between or adjacent other layers of the bridge 322. The absorbent layer 380, which may include a super-absorbent material, may enable or enhance the ability of the bridge 222 to store fluid, such as wound exudates from a tissue site.

Figure 4 illustrates a cross-section view of an example embodiment of one layer of manifold material, such as the first manifold layer 350. In some embodiments, the acquisition side 360a may be comprised of vertical fibers 420, and the distribution side 362a may be comprised of longitudinal fibers 422. The longitudinal fibers 422 may be oriented substantially in a longitudinal direction along the length of the first manifold layer 350, which may largely correspond to the length of the bridge 222. The vertical fibers 420 may be oriented substantially vertical or normal relative to the longitudinal fibers 422 and the length of the first manifold layer 350 and the bridge 222. The distribution side 362a may be coupled to the acquisition side 360a. Fluid communication voids 424 may be located or defined between and among the longitudinal fibers 422 of the distribution side 362a and the vertical fibers 420 of the acquisition side 360a. The fluid communication voids 424 may provide fluid communication through the first manifold layer 350 of the manifold material even when exposed to a force, such as compression force depicted in Figure 4 as arrows 426, for example. When exposed to such a force, the longitudinal fibers 422 and the vertical fibers 420 may engage one another to substantially preclude blockage, closure, or other interference with the fluid communication voids 424 in providing fluid communication through the first manifold layer 350 of the manifold material, as well as the overall bridge 222.

During operation of the therapy system 100, the negative-pressure source 102 may be activated to provide negative pressure to the dressing 104. For example, in some of the embodiments employing the bridge 222, negative pressure may be provided to the first end 224 of the bridge 222. The negative pressure may be transmitted through the layers of manifold material in the internal passageway provided by the sealing member. The negative pressure may be further transmitted through the second end 226 of the bridge 222 and into the dressing 104 and to a tissue site.

Negative pressure can be transmitted to the dressing 104 and tissue site to draw, wick, or pull fluid from the tissue site into the dressing 104, and further into the bridge 222. As fluid enters the bridge 222 through the second end 226, the fluid may be moved through the second aperture 332 and contact the fluid acquisition side 360a of the first manifold layer 350. The fluid acquisition side 360a of the first manifold layer 350 may receive the fluid so that the fluid may be transported through the first manifold layer 350. Subsequently, the fluid distribution side 362a of the first manifold layer 350 may transmit some of the fluid along the length of the first manifold layer 350 and bridge 222 within the internal passageway to the first end 224. The fluid distribution side 362a of the first manifold layer 350 may also transmit some portion of the fluid to the fluid acquisition side 360b of the second manifold layer 352.

As fluid is transmitted from the fluid distribution side 362a of the first manifold layer 350, the fluid may contact at least a peripheral portion of the fluid acquisition side 360b of the second manifold layer 352. The fluid acquisition side 360b of the second manifold layer 352 may receive the fluid so that the fluid may be transported through the second manifold layer 352. Subsequently, the fluid distribution side 362b of the second manifold layer 352 may transmit the fluid directly to the first end 224 of the bridge 222. However, in some embodiments, the fluid distribution side 362b of the second manifold layer 352 may also transmit fluid to a fluid acquisition side 360c of the third manifold layer 354. The fluid may thus also be transported through the third manifold layer 354 along a third distribution side 362c of the third manifold layer 354 towards the first end 224 of the bridge 222.

As fluid is moved along the length of the bridge 222 by the one or more manifold layers, as well as between the manifold layers, such as the first manifold layer 350, the second manifold layer 352, and the third manifold layer 354, at least some amount of the fluid may pass along or through the binding layer 370. As the fluid passes along or through the binding layer 370, the binding material of the binding layer 370 may bind proteins as well as bacteria, particularly when the fluid possesses a higher viscosity due to a high concentration of proteins or bacteria. As proteins and bacteria are bound by the binding layer 370, the remaining components of the fluid may continue to be transported along or through the manifold layers. Rather than the highly-viscous protein components of the fluid becoming saturated in the one or more manifold layers and thus impairing or preventing the transmission of the fluid, as well as negative pressure, through and along the manifold layers, the proteins may remain bound to or within the binding layer 370. The remaining fluid components may continue to be transported through the bridge 222. With the improved fluid flow provided by the inclusion of the binding layer 370, fluid may be better transmitted via the one or more manifold layers along the length of the bridge 222. Additionally, more effective evaporation of the fluid, in the form of vapor, through the portions of the sealing member, such as the second sealing layer 344, may be maintained throughout the duration of the therapy system 100 being applied to a patient.

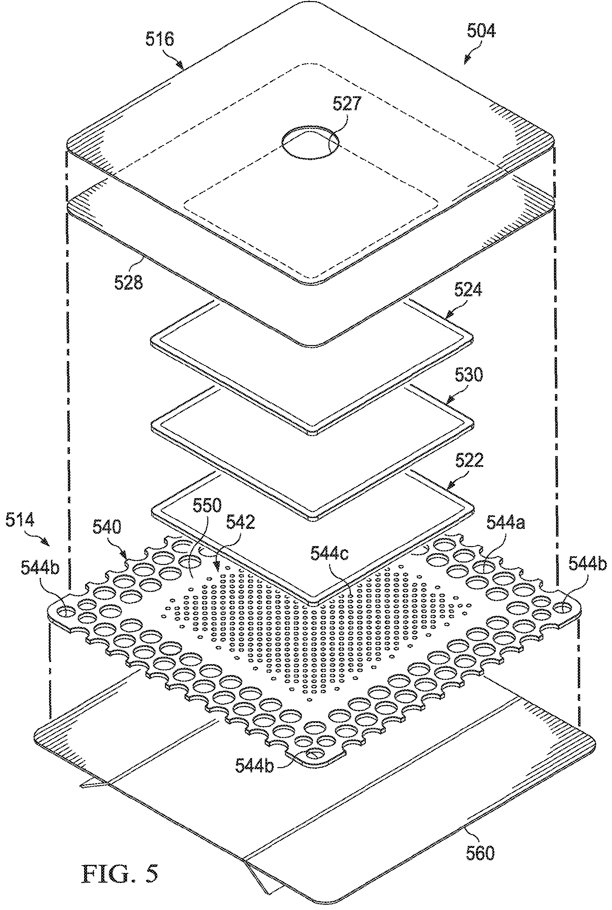

Figure 5 illustrates features of another illustrative embodiment of a dressing, dressing 504. Dressing 504 may include a tissue interface 514 and a cover 516, as well as additional manifold layers and a binding material for binding bacteria and/or proteins. For example, in some embodiments, the dressing 504 may include a tissue interface 514, a first manifold layer 522, a second manifold layer 524, a cover 516, and an adhesive 528. The dressing 504 may further include a binding layer 530. Components of the dressing 504 may be added or removed to suit a particular application.

In some embodiments, the tissue interface 514 may have a periphery 540 surrounding a central portion 542, and a plurality of apertures 544 disposed throughout the periphery 540 and the central portion 542. The tissue interface 514 may also have a border 550 substantially surrounding the central portion 542 and positioned between the central portion 542 and the periphery 540. The border 550 may be free of the apertures 544. The tissue interface 514 may be adapted to cover the tissue site as well as the tissue surrounding the tissue site, such that the central portion 542 of the tissue interface 514 is positioned adjacent to or proximate to the tissue site, and the periphery 540 is positioned adjacent to or proximate to tissue surrounding the tissue site. Further, the apertures 544 in the tissue interface 514 may be in fluid communication with the tissue site and tissue surrounding the tissue site. In some embodiments, the dressing 504 may further include an additional structure for placement against or within the tissue site, such as a wound filler.

The apertures 544 in the tissue interface 514 may have any shape, such as for example, circles, squares, stars, ovals, polygons, slits, complex curves, rectilinear shapes, triangles, or other shapes. The apertures 544 may be formed by cutting, by application of local RF energy, or other suitable techniques for forming an opening. As shown in Figure 5, each of the plurality of apertures 544 may be substantially circular in shape. Each of the plurality of apertures 544 may have an area, which may refer to an open space or open area defining each of the plurality of apertures 544. The area of each of the plurality of apertures 544 may be substantially the same, or the areas of individual apertures of the plurality of apertures 544 may vary depending, for example, on the position of the individual aperture in the tissue interface 514. For example, the area of the apertures 544 in the periphery 540 may be larger than the area of the apertures 544 in the central portion 542 of the tissue interface 514. The plurality of apertures 544 may have a uniform pattern or may be randomly distributed on the tissue interface 514. The size and configuration of the plurality of apertures 544 may be designed to control the adherence of the cover 516 to an epidermis surrounding a tissue site.

In some embodiments, the plurality of apertures 544 positioned in the periphery 540 of the tissue interface 514 may be apertures 544a. Additionally, the plurality of apertures 544 positioned at corners of the periphery 540 of the tissue interface 514 may be apertures 544b. Furthermore, the plurality of apertures 544 positioned in the central portion 542 of the tissue interface 514 may be apertures 544c. Each of the apertures 544a-544c may vary in size. However, in some embodiments, the apertures 544a may have a diameter between about 9 millimeters to about 11 millimeters. The apertures 544b may have a diameter between about 7 millimeters to about 9 millimeters. The apertures 544c may have a diameter between about 1.5 millimeters to about 3 millimeters. Furthermore, the spacing between each of the apertures 544a-c may also vary depending on the specific embodiment. For example, in some embodiments, the diameter of each of the apertures 544a may be separated from one another by a distance of between about 2.5 millimeters to about 3.5 millimeters. Further, the diameter of at least one of the apertures 544a may be separated from the diameter of at least one of the apertures 544b by approximately a distance of about 2.5 millimeters to about 3.5 millimeters. The diameter of each of the apertures 544b may also be separated from one another by a similar distance. Additionally, a center of one of the apertures 544c may be separated from a center of another of the apertures 544c in a first direction by a distance of between about 2.5 millimeters to about 3.5 millimeters. In a second direction transverse to the first direction, the center of one of the apertures 544c may be separated from the center of another of the apertures 544c by a distance of between about 2.5 millimeters to about 3.5 millimeters. As shown in Figure 5, the distances may be increased for the apertures 544c in the central portion 542 being positioned proximate to or at the border 550 as compared to the apertures 544c positioned away from the border 550.

As shown in Figure 5, the central portion 542 of the tissue interface 514 may be substantially square with each side of the central portion 542 having a length of between about 100 millimeters to about 150 millimeters. In some embodiments, the length may be between about 100 millimeters to about 110 millimeters. The dimensions of each section of the tissue interface 514, such as the periphery 540, the center portion 542, and the border 550 may vary based on the particular application of the dressing 504.

The tissue interface 514 may be a soft, pliable material suitable for providing a fluid seal with a tissue site. For example, the tissue interface 514 may comprise a silicone gel, a soft silicone, hydrocolloid, hydrogel, polyurethane gel, polyolefin gel, hydrogenated styrenic copolymer gel, a foamed gel, a soft closed-cell foam such as polyurethanes and polyolefins coated with an adhesive, polyurethane, polyolefin, or hydrogenated styrenic copolymers. In some embodiments, the tissue interface 514 may be perforated silicone layer or a non-adherent polyurethane or polyethylene film. The tissue interface 514 may also be an ethylene vinyl acetate (EVA) mesh. The tissue interface 514 may have a thickness between about 500 micrometers and about 1,000 micrometers. Further, in some embodiments, the tissue interface 514 may be comprised of hydrophobic or hydrophilic materials.

In some embodiments (not shown), the tissue interface 514 may be a hydrophobic-coated material. For example, the tissue interface 514 may be formed by coating a spaced material, such as, for example, woven, nonwoven, molded, or extruded mesh with a hydrophobic material. The hydrophobic material for the coating may be a soft silicone, for example.

The adhesive 528 may be in fluid communication with the plurality of apertures 544 in at least the periphery 540 of the tissue interface 514. In this manner, the adhesive 528 may be in fluid communication with tissue surrounding a tissue site through the plurality of apertures 544 in the tissue interface 514. The adhesive 528 may extend or be passed through the plurality of apertures 544 to contact epidermis for securing the cover 516 to, for example, tissue surrounding a tissue site. The plurality of apertures 544 may provide sufficient contact of the adhesive 528 to the epidermis to secure the cover 516 about a tissue site. The plurality of the apertures 544 and the adhesive 528 may also be configured to permit release and repositioning of the cover 516 about a tissue site.

In some embodiments, an additional or alternative attachment device may be used to secure the cover 516 about the tissue site. For example, double-sided tape, paste, hydrocolloid, hydrogel, silicone gel, or organogel may be used. Furthermore, thicker adhesives, or combinations of adhesives, may be applied in some embodiments to improve seals and to reduce leaks. Additionally, any of the plurality of the apertures 544 may be adjusted in size and number to maximize the surface area of the adhesive 528 in fluid communication through the plurality of the apertures 544 for a particular application or geometry of the tissue interface 514.

The adhesive 528 may be a medically-acceptable adhesive. The adhesive 528 may also be flowable. For example, the adhesive 528 may comprise an acrylic adhesive, rubber adhesive, high-tack silicone adhesive, polyurethane, or other adhesive substance. In some embodiments, the adhesive 528 may be a pressure-sensitive adhesive, such as an acrylic adhesive with coating weight of 15 grams/m² (gsm) to 70 grams/m2 (gsm). The adhesive 528 may be a layer having substantially the same shape as the periphery 540 of the tissue interface 514, and thus have a large central aperture, as shown in Figure 5. In some embodiments, the layer of the adhesive 528 may be continuous or discontinuous. Discontinuities in the adhesive 528 may be provided by apertures (not shown) in the adhesive 528. Apertures in the adhesive 528 may be formed after application of the adhesive 528 or by coating the adhesive 528 in patterns on a carrier layer, such as, for example, a side of the cover 516 adapted to face the epidermis. Further, apertures in the adhesive 528 may be sized to control the amount of the adhesive 528 extending through the plurality of the apertures 544 in the tissue interface 514 to reach the epidermis. Apertures in the adhesive 528 may also be sized to enhance the Moisture Vapor Transfer Rate (MVTR) of the cover 516, described in further detail below.

Factors that may be utilized to control the adhesion strength of the cover 516 may include the diameter and number of the plurality of the apertures 544 in the tissue interface 514, the thickness of the tissue interface 514, the thickness and amount of the adhesive 528, and the tackiness of the adhesive 528. An increase in the amount of the adhesive 528 extending through the plurality of the apertures 544 may correspond to an increase in the adhesion strength of the cover 516. A decrease in the thickness of the tissue interface 514 may correspond to an increase in the amount of adhesive 528 extending through the plurality of the apertures 544. Thus, the diameter and configuration of the plurality of the apertures 544, the thickness of the tissue interface 514, and the amount and tackiness of the adhesive 528 utilized may be varied to provide a desired adhesion strength for the cover 516. For example, in some embodiments, the thickness of the tissue interface 514 may be about 200 micrometers, the adhesive 528 may be a layer having a thickness of about 30 micrometers and a tackiness of 2000 grams per 25 centimeter wide strip, and the diameter of the apertures 544a in the tissue interface 514 may be about 10 millimeters.

Still referring primarily to Figure 5, a release liner 560 may be attached to or positioned adjacent to the tissue interface 514 to protect the adhesive 528 prior to application of the dressing 504 to the tissue site. Prior to application of the dressing 504 to the tissue site, the tissue interface 514 may be positioned between the cover 516 and the release liner 560. Removal of the release liner 560 may expose the tissue interface 514 and the adhesive 528 for application of the dressing 504 to the tissue site. The release liner 560 may also provide stiffness to assist with, for example, deployment of the dressing 504. The release liner 560 may be, for example, a casting paper, a film, or polyethylene. Further, the release liner 560 may be a polyester material such as polyethylene terephthalate (PET), or similar polar semicrystalline polymer. A release agent may be disposed on a side of the release liner 560 that is configured to contact the tissue interface 514. For example, the release agent may be a silicone coating and may have a release factor suitable to facilitate removal of the release liner 560 by hand and without damaging or deforming the dressing 504. In some embodiments, the release agent may be fluorosilicone. In other embodiments, the release liner 560 may be uncoated or otherwise used without a release agent.

The peripheral portions of the cover 516 may be positioned proximate to the periphery 540 of the tissue interface 514 such that a central portion of the cover 516 and the central portion 542 of the tissue interface 514 define an enclosure. The adhesive 528 may be positioned at least between the peripheral portions of the cover 516 and the periphery 540 of the tissue interface 514. The cover 516 may cover the tissue site and the tissue interface 514 to provide a fluid seal and a sealed space between the tissue site and the cover 516. Further, the cover 516 may cover other tissue, such as a portion of epidermis, surrounding the tissue site to provide the fluid seal between the cover 516 and the tissue site. In some embodiments, a portion of the peripheral portion of the cover 516 may extend beyond the periphery 540 and into direct contact with tissue surrounding the tissue site. In some embodiments, the peripheral portion of the cover 516, for example, may be positioned in contact with tissue surrounding the tissue site to provide the sealed space without the tissue interface 514. Thus, the adhesive 528 may also be positioned at least between the peripheral portion of the cover 516 and tissue, such as the epidermis, surrounding the tissue site. The adhesive 528 may be disposed on a surface of the cover 516 adapted to face the tissue site and the tissue interface 514. Additionally, the cover 516 may include an aperture 527, which in some embodiments may be generally positioned in a central portion of the cover 516. The aperture 527 may allow for fluid communication between a sealed space provided by the cover 516 and including a tissue site, and a conduit for conducting negative pressure, such as the bridge 222.

The cover 516 may be formed from any material that allows for a fluid seal, such as any of the materials of the cover 116. The cover 516 may be vapor permeable and liquid impermeable, thereby allowing vapor and inhibiting liquids from exiting the sealed space provided by the cover 516. In some embodiments, the cover 516 may be a flexible, breathable film, membrane, or sheet having a high MVTR and other properties similar to those described with respect to the cover 116. In other embodiments, a low or no vapor transfer drape might be used. In some embodiments, the cover 516 may comprise a range of medically suitable films having a thickness between about 15 microns (µm) to about 50 microns (µm).

The dressing 504 may include one or more layers of a manifold material positioned between the tissue interface 514 and the cover 516. For example, the dressing 504 may include a first manifold layer 522 and a second manifold layer 524. In some embodiments, the dressing 504 may include additional layers of manifold material, for example, a third manifold layer and a fourth manifold layer. In additional embodiments, the dressing 504 may include only one layer of a manifold material. Similarly to the manifold material described with respect to the bridge 222 of Figures 2-3, the manifold material may include a wicking material. Furthermore, the one or more manifold layers may include a non-woven material, such as, for example, a polyester non-woven or Libeltex TDL4 material, and any of the other materials previously discussed with respect to the manifold material of bridge 222. In some embodiments, other non-woven materials may be used for the manifold material, such as Libeltex TDL2 material, or laminations with fiber or foam structures.

The binding layer 530 may include a binding material which may demonstrate bacterial-binding as well as protein-binding properties, similar to the binding material of the binding layer 370 of the bridge 222 discussed above. As depicted in Figure 5, in some embodiments, the binding layer 530 may be generally in the form of a sheet and may be positioned between two or more layers of manifold material within the structure of the dressing 504. For example, the binding layer 530 may be disposed between the first manifold layer 522 and the second manifold layer 524. Similar to the function of the binding layer 370 discussed above with respect to Figure 3, the binding layer 530 may assist with binding bacteria as well as protein material that may be drawn or wicked out of a tissue site, such as a wound, through components of the dressing 504.

In some embodiments, the dressing 504 may include additional components or layers, such as, for example, an absorbent. An example absorbent may comprise or consist of a hydrophilic material adapted to absorb fluid, for example from a tissue site. The absorbent may include, without limitation, any number of individual absorbent components as desired for treating a particular tissue site, including but not limited to superabsorbent materials. For example, the dressing 504 may further include a layer of superabsorbent material, which may be positioned between the one or more layers of manifold material, such as the first manifold layer 522 and the second manifold layer 524. In some embodiments of the therapy system 100, the container 106 may be omitted, and it may be particularly beneficial or necessary to include a structure having absorbent capabilities in the dressing 504 or in a fluid conductor of the therapy system 100, such as the bridge 122. The dressing 504 may also include other additional layers, such as additional wicking or manifolding layers, based on the specific needs or application of the dressing 504.

As the dressing 504 comes into contact with fluid from a tissue site, the fluid may come into contact with the tissue interface 514. The fluid may then pass through the apertures 544 of the tissue interface 514 toward the first manifold layer 522. The first manifold layer 522 may wick or otherwise move the fluid through the tissue interface 514 and away from the tissue site. The tissue interface 514 may be adapted to transfer fluid away from a tissue site rather than store the fluid. For example, in some embodiments the first manifold layer 522 may have a higher affinity for fluid than the tissue interface 514. As fluid, such as wound exudate, is drawn away from a tissue site, the fluid may pass through the tissue interface 514 in response to a wicking force generated by the manifold material, such as the first manifold layer 522 and the second manifold layer 524, as well as due to the application of negative pressure to the dressing 504. Fluid in the first manifold layer 522 and the second manifold layer 524 may be drawn against, along, or through the binding layer 530. The binding material of the binding layer 530 may bind bacteria and proteins in the fluid, while allowing remaining components of the fluid to continue to pass through the first manifold layer 522, binding layer 530, and second manifold layer 524 towards the cover 516. Once the fluid reaches the cover 516, the fluid may be drawn through the aperture 527 in the cover 516, out of the dressing 504, and into a fluid conductor, such as a bridge 122, and more specifically the bridge 222 of Figures 2-3. Additionally, vapor may be evaporated through the cover 516 and into the environment external to the dressing 504.

Additional embodiments of the therapy system 100 may also be provided, in which material for binding bacteria and/or protein may be applied to one or more other components of the therapy system 100, in addition to or instead of the binding layer 370 of the bridge 222 or the binding layer 530 of the dressing 504. In some embodiments of the therapy system 100, instead of introducing an additional binding layer to either the bridge 122 or the dressing 504, a binding material, such as a DACC material, as previously discussed, may be applied to or coated on one or more different components or structures of the therapy system 100. For example, a coating of binding material may be applied to one or more components of the dressing 504, such as the tissue interface 514, manifold material, such as the first manifold layer 522 and the second manifold layer 524, and an inside surface of the cover 516. A binding material coating may additionally or alternatively be applied to one or more components of the bridge 222, including but not limited to the first sealing layer 342 and the second sealing layer 344 or the one or more layers of manifold material, such as the first manifold layer 350, the second manifold layer 352, and the third manifold layer 354. The binding material may also be coated on an optional layer of absorbent material which may be included in either or both of the bridge 222 or the dressing 504.

Additionally, in some embodiments of the therapy system 100, one or more fluid conductors between the bridge 122 and other components of the therapy system 100, such as the negative-pressure source 102 or container 106, may be coated on an interior surface with a binding material, such as the DACC material. Coating a fluid conductor with a binding material, such as DACC material, may provide the additional benefit of preventing microorganism growth within fluid that may be in the fluid conductor or stored within the container 106.

Some embodiments of the therapy system 100 may also include the use of additional antiseptic materials in addition to or in lieu of the DACC material. For example, one or more layers of either the dressing 104 or the bridge 122 may be coated or bound with an antiseptic material, such as polyhexanide (PHMB) or activated charcoal. Additionally, the use of inherently "active" antimicrobial materials such as silver, copper, zinc, or titanium, may be appropriate. For example, in addition to or instead of a binding material coating, a coating of antimicrobial material, such as a silver-containing compound, may be applied to any of the above-mentioned layers and components of the bridge 222 and dressing 504. In some embodiments, active antimicrobial materials may be included in components of the therapy system 100 which are further removed from communication with a tissue site, such as by including the active materials within the bridge 222, so as to minimize any inadvertent effects of active antimicrobial agents to the healing of the tissue site.

The therapy system 100 may be supplied as a kit. In some embodiments, a kit may include a dressing 104 and a bridge 122. A negative-pressure source 102 may also be provided in conjunction with the kit. The dressing 104 and the bridge 122 may be conveniently applied by a nurse or other caregiver. In some embodiments, the kit may include a bridge 122 fluidly pre-connected to a dressing 104. Additionally, a kit may include an interface, such as conduit interface 336 and a tubeset that may be supplied attached or unattached to the interface, such as conduit interface 336. In some instances, the tubeset may allow for connection to more than one type of negative-pressure source, such as negative-pressure source 102.

The systems, apparatuses, and methods described herein may provide significant advantages. For example, the therapy system 100 may provide a discrete, low-profile, fluid-managing negative-pressure dressing with a bacterial-binding material that can prevent the migration of bacteria back to a tissue site and possible reinfection. Further, incorporating a binding material as part of the bridge 122 may be particularly advantageous for retaining any bacterial colonization within the bridge 122 and maximizing the distance between bound bacteria and a tissue site. Furthermore, the benefits of including binding materials, such as DACC material, may offer significant benefits associated with binding bacteria and proteins, without incorporating active antimicrobials, such as silver, copper, zinc, or titanium, within the dressing structure that interfaces with a tissue site, and can reduce or minimize side effects of active antimicrobial agents. The therapy system 100 also provides for a number of optional configurations, including adding additional functional layers or components to either or both of the dressing 104 or bridge 122. A super-absorbent component may further augment the bacterial- and protein-binding potential of the dressing 104 and/or bridge 122.

As previously discussed, the incorporation of the binding material into the components of the therapy system 100, such as the dressing 104 or bridge 122, may offer significant benefits due to the ability of the binding material to surprisingly bind proteins in wound exudate. Unexpectedly, binding material such as the DACC material, for example the SORBACT product, binds proteins in wound exudate. The binding of the proteins may offer significant improvements to the evaporative and fluid management capabilities of the dressing 104 and bridge 122, for example, due to reduction of the potential occlusion by viscous wound exudate caused by the high concentrations of proteins. The binding of proteins in wound exudate by the binding material may help preserve the ability of the other components of a dressing 104 or bridge 122 to communicate negative pressure and transport fluid. By including the binding material, therapy system 100 may be better able to conduct negative pressure to a tissue site as well as transport wound exudate away from a tissue site, even after multiple days of being applied to a tissue site that is exuding highly-viscous fluid. By maintaining good communication of negative pressure within the components of the therapy system 100, such as the dressing 104 and the bridge 122, the inclusion of the binding material may help prevent occlusions due to high protein content of viscous wound fluid within the system components and the corresponding pressure drops across different components. For example, the inclusion of the binding material in the binding layer 370 of the bridge 222 may prevent pressure drops across the length of the bridge 222, which may otherwise occur with the use of multiple layers of low-profile manifold or wicking materials alone, such as the first manifold layer 350, the second manifold layer 352, and the third manifold layer 354. Furthermore, the binding layer 370 may offer a lower-profile approach to improve the fluid management capabilities of the bridge 222 without adding significant empty volume to the bridge 222, which may otherwise result from previous solutions involving adding more layers of non-woven material or foam to try to improve fluid management and avoid blockages. Thus, the incorporation of the low-profile binding layer 370 in the bridge 222 may improve fluid management capabilities, without significantly increasing volume in the bridge 222 that a mechanical negative-pressure therapy source has to evacuate and maintain at a set level of negative pressure.

As also previously discussed, the therapy system 100 can also provide particular advantages for treating some forms of specialized tissue sites, such as ulcers, and more particularly VLUs. VLUs are typically specialized, shallow wounds that occur on the lower leg just above the ankle and tend to affect older patient populations. Current standards of care for treating VLUs often prescribe the use of a simple, non-adherent dressing covered by a compression bandage, with the aim of improving blood flow in the legs of the patient. The dressing and/or compression bandage is often recommended to be changed every seven days by a trained clinician or caregiver. In many instances, the VLU may take anywhere from four to six weeks to heal under such treatment conditions.

It would be advantages, in many cases, to provide VLUs with the benefits of negative-pressure wound therapy to assist with controlling wound exudate, encourage blood flow, and promote healing. However, it may also be important to provide such negative-pressure therapy while allowing the patient to remain ambulatory and maintain a normal lifestyle of day-to-day activities between dressing changes. In fact, in many instances of VLUs, patient mobility is encouraged during treatment to prevent additional patient comorbidities, however this can be challenging given the debilitating nature of VLUs. Thus, considering these factors, it would be beneficial for such a negative-pressure therapy device or system for treatment of VLUs to be lightweight and portable, while also allowing for discrete use in public settings. For example, the negative-pressure source 102 may include a negative-pressure therapy device that may be particularly applicable for use with the therapy system 100 for treatment of VLUs, such as the SNAP^{™} Therapy Cartridge, available from Acelity of San Antonio, Texas, or the NANOVA^{™} Therapy Unit, available from KCI of San Antonio, Texas. Both of these negative-pressure devices may be configured to be highly-portable, mechanical devices without the need for an electrical power source. However, given the small size and portability of such negative-pressure therapy devices, it may be particularly important to pair these devices with one or more dressing and/or system components that are designed for minimizing pressure differentials or drops for managing fluid properly and efficiently. For example, components of the dressing 104, as well as components in the fluid conductors of the therapy system 100, such as the bridge 222, may be incorporated to reduce or prevent occlusions or blockages in order to maintain continuous transmission of negative pressure through the therapy system 100.

Depending on the particular type of negative-pressure source 102 included in the therapy system 100, different combinations and variations of the dressing 104 and the bridge 122 may be incorporated into the therapy system 100. For example, in embodiments of the therapy system 100 that include a SNAP^{™} cartridge, it may be beneficial to use both a dressing 104 and bridge 122 that both omit a separate absorbent layer, such as the absorbent layer 380 of Figure 3B. In other embodiments of the therapy system 100 that include a NANOVA^{™} unit, either or both of the dressing 104 and bridge 122 may incorporate an absorbent layer, such as the absorbent layer 380 of Figure 3B.

Moreover, distribution components such as the dressing 104 and the bridge 122 may have a low profile for discrete use with a shallow wound such as a VLU, and can minimize pressure drops. The dressing 104 and the bridge 122 may enable the use of a lightweight, portable negative-pressure therapy device, such as a SNAP^{™} cartridge or NANOVA^{™} unit, for providing the required level of negative pressure to a tissue site for an extended period. The dressing 104 may also have a low-profile suitable for use under a compression garment. Thus, some embodiments of the dressing 104 and the bridge 122 may be particularly suitable for use with a 7-day compression bandage treatment regime for VLUs. The compression garment, bandage, or stocking may either be used to cover both the bridge 122 and the dressing 104 or just the dressing 104 itself. Depending on the particular application, the compression garment, bandage, or stocking should also be breathable so as not to prevent the exchange of air flow with the bridge 122 and/or dressing 104 that may be required to allow the bridge 122 and/or dressing 104 to provide the beneficial fluid management and vapor evaporation functionalities. The compression garment may also contain an antimicrobial element. The dressing 104 and the bridge 122, due to the incorporation of a binding material, should also be capable of effectively mitigating and combating the propensity for bacterial growth that can occur in dressings worn for longer durations, such as those worn for up to 7 days.

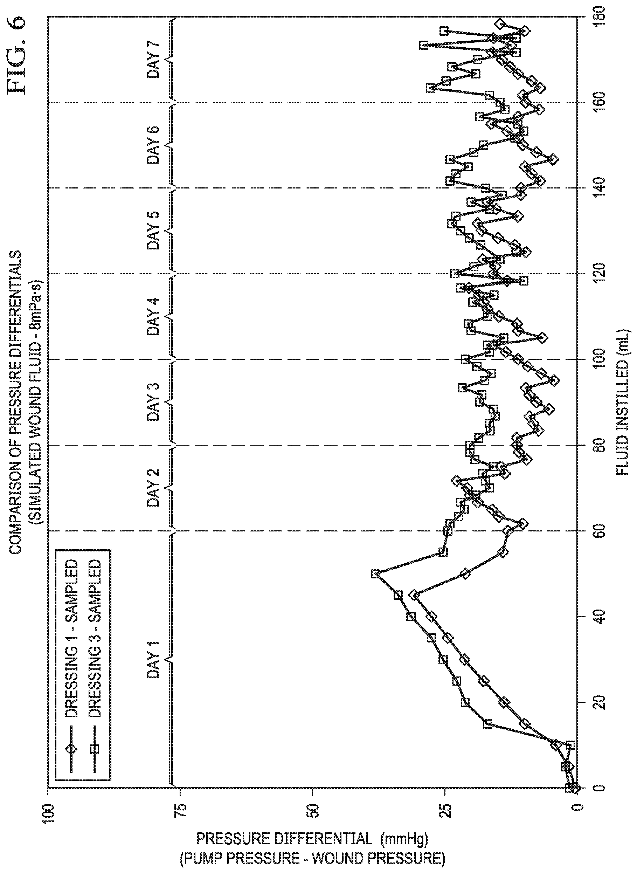

Beneficial effects of incorporating the binding material in one or more components of the therapy system 100 may be illustrated in part by Figures 6-8. For example, Figure 6 provides a chart comparing the performance of two different bridge dressings at minimizing pressure drops across each of the bridge dressings. Each of the bridge dressings was applied to a simulated tissue site and subjected to a seven day test period during which fluid was instilled, in order to simulate conditions of wound exudates at a tissue site. A first bridge dressing included in the experiment, designated as Dressing 1, included one binding layer comprising binding material disposed between three layers of wicking material, which in this instance were three layers of Libeltex TDL2 80gsm material. The binding layer of Dressing 1 included an acetate fabric impregnated with DACC with an enclosed gauze layer, such as the Cutimed^{®} Sorbact^{®} product. A second bridge dressing in the experiment, designated as Dressing 3, included four layers of wicking material, which were also the Libeltex TDL2 80gsm material. Dressing 3 did not include a binding layer.

The two dressings, Dressing 1 and Dressing 3, were each tested according to a protocol where simulated wound fluid having a viscosity of approximately 8 mPa·s was instilled to a simulated wound over a period of 7 days. The simulated wound fluid was applied using a calibrated syringe driver. The flow rate of the simulated wound fluid on Day 1 was 60 mL / 24 hours, and the flow rate for Days 2-7 was 20 mL / 24 hours. The 8 mPa·s simulated wound fluid included the components as listed in Table 1, below.

**Table 1. Ingredients Table of 8 mPa·s Simulated Wound Fluid By Batch Size (L)**

| | **1 (L)** | **2 (L)** | **3 (L)** | **4 (L)** | **5 (L)** | **6 (L)** | **7 (L)** | **8 (L)** | **9 (L)** | **10 (L)** |
|---|---|---|---|---|---|---|---|---|---|---|
| **Deionized water (L)** | **0.938** | **1.87** | **2.805** | **3.74** | **4.675** | **5.61** | **6.545** | **7.48** | **8.415** | **9.35** |
| **Albumin (g)** | **0.867** | **1.735** | **2.603** | **3.47** | **4.338** | **5.205** | **6.073** | **6.94** | **7.808** | **8.675** |
| **Cosmedia (g)** | **2.07** | **4.14** | **6.21** | **8.28** | **10.35** | **12.42** | **14.49** | **16.56** | **18.63** | **20.7** |
| **PBS (mL)** | **65** | **130** | **195** | **260** | **325** | **390** | **455** | **520** | **585** | **650** |
| **Amino Acids (µL)** | **18.75** | **37.5** | **56.25** | **75** | **93.75** | **112.5** | **131.25** | **150** | **168.75** | **187.5** |
| **Glucose (mg)** | **56.25** | **112.5** | **168.75** | **225** | **281.25** | **337.5** | **393.75** | **450** | **506.25** | **562.5** |
| **Food coloring (mL)** | **0.08** | **0.16** | **0.24** | **0.32** | **0.4** | **0.48** | **0.56** | **0.64** | **0.72** | **0.8** |

Each of Dressing 1 and Dressing 3 was incorporated within a simulated therapy system to model the therapy system 100, where each simulated system included at least a dressing for placing over the simulated wound, a low-profile conduit or bridge dressing (Dressing 1 or Dressing 3), and a negative-pressure source. Any additional connectors, interfaces, or tubing necessary to complete the simulated therapy system were used consistently between the two systems testing Dressing 1 and Dressing 3. Negative pressure was applied by the same form of negative-pressure source capable of delivering a consistent amount of negative pressure at the pump, which may otherwise be referred to as the pump pressure. For example, in this experiment, an INFOV.A.C.^{™} Therapy Unit, commercially available from KCI of San Antonio, TX, was used to generate negative pressure at a constant -125mmHg. The negative pressure was delivered at -125 mmHg for a constant 7-day period. During this 7-day test period, pressure measurements, such as the pressure differential across each of the lengths of the two tested bridge dressings, Dressing 1 and Dressing 3, were measured at standard time intervals, taking instantaneous measurements. In this experiment, the pressure differential across the length of each bridge dressing was determined by subtracting the pressure level measured at the simulated wound from the pump pressure which in this instance was set to a controlled negative pressure of - 125 mmHg (Pressure Differential = Pump Pressure - Wound Pressure).

As evident from the chart of Figure 6, there is a notable reduction in pressure differential, and thus improvement in the ability to communicate negative pressure, of Dressing 1 as compared to Dressing 3. Results indicated that the average pressure drop or differential, which may be considered in absolute values, was only 12.94 mmHg (Std. Dev. of 5.73) over the 7-day period for Dressing 1, which included the binding layer of Cutimed^{®} Sorbact^{®} DACC material sandwiched between the three layers of Libeltex TDL2 80gsm material. In contrast, the average pressure drop was 19.39 mmHg (Std. Dev. of 5.80) over the same 7-day period for Dressing 3, which included the four layers of Libeltex TDL2 80gsm material, and no binding layer. Thus, an approximate 35% difference in pressure drop was realized between Dressing 1 and Dressing 3, which can be attributed to the inclusion of the binding layer in Dressing 1.

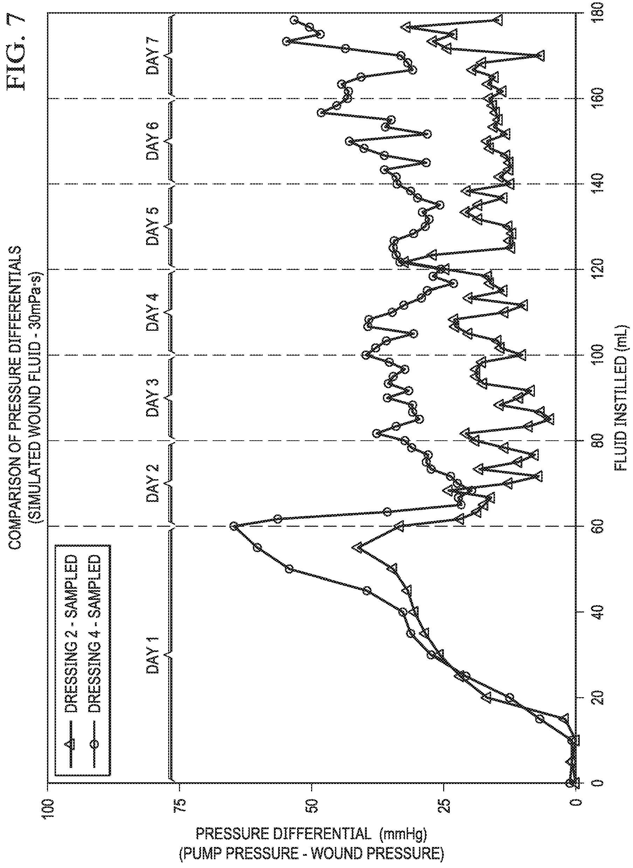

Figure 7 includes a chart, similar to that of Figure 6, showing the results of an experiment comparing the performance of two different bridge dressings. However, in the experiment of Figure 7, the two tested bridge dressings were tested using simulated wound fluid having a significantly higher viscosity of approximately 30 mPa·s. The first bridge dressing included in the experiment of Figure 7, designated as Dressing 2, included one binding layer of Cutimed^{®} Sorbact^{®} material sandwiched between three layers of manifold material, which in this instance were three layers of Libeltex TDL2 80gsm material. The second bridge dressing in this experiment, designated as Dressing 4, included four layers of manifold material, once again the Libeltex TDL2 80gsm material. Dressing 4 did not include a binding layer.

Once again, the two dressings, Dressing 2 and Dressing 4, were tested according to the same testing protocols as the experiment of Dressing 1 and Dressing 3, as described in relation to Figure 6. However, as already noted, the simulated wound fluid in the experiment of Figure 7 had a viscosity of approximately 30 mPa·s, as compared to the simulated wound fluid of the experiment of Figure 6, which had a viscosity of approximately 8 mPa·s. To achieve the higher viscosity of approximately 30 mPa·s, the simulated wound fluid of the experiment of Figure 7 was prepared with a greater amount of the albumin protein and Cosmedia (thickening agent) components. The 30 mPa·s simulated wound fluid included the components as listed in Table 2, below.

**Table 2. Ingredients Table of 30 mPa·s Simulated Wound Fluid By Batch Size (L)**

| | **1 (L)** | **2 (L)** | **3 (L)** | **4 (L)** | **5 (L)** | **6 (L)** | **7 (L)** | **8 (L)** | **9 (L)** | **10 (L)** |
|---|---|---|---|---|---|---|---|---|---|---|
| **Deionized water (L)** | **0.938** | **1.88** | **2.81** | **3.75** | **4.69** | **5.63** | **6.57** | **7.50** | **8.44** | **9.38** |
| **Albumin (g)** | **2.22** | **4.44** | **6.66** | **8.88** | **11.1** | **13.32** | **15.54** | **17.76** | **19.98** | **22.2** |
| **Cosmedia (g)** | **5.30** | **10.6** | **15.9** | **21.2** | **26.5** | **31.8** | **37.1** | **42.4** | **47.7** | **53** |
| **PBS (mL)** | **100** | **200** | **300** | **400** | **500** | **600** | **700** | **800** | **900** | **1000** |
| **Amino Acids (µL)** | **48.0** | **96.0** | **144** | **192** | **240** | **288** | **336** | **384** | **432** | **480** |
| **Glucose (mg)** | **144** | **288** | **432** | **576** | **720** | **864** | **1008** | **1152** | **1296** | **1440** |
| **Food coloring (mL)** | **0.08** | **0.16** | **0.24** | **0.32** | **0.40** | **0.48** | **0.56** | **0.64** | **0.72** | **0.80** |

As shown in the chart of Figure 7, a significant reduction in pressure differential of Dressing 2 is seen as compared to Dressing 4, which does not include the binding layer, but rather only manifold layers. More specifically, the average pressure drop was 17.39 mmHg (Std. Dev. of 7.67) over the 7-day test period for Dressing 2, as opposed to the average pressure drop of 33.89 mmHg (Std. Dev. of 11.43) for Dressing 4. Accordingly, an approximate 50% difference in pressure drop was realized between the two tested dressings, which can be attributed to the inclusion of the binding layer in Dressing 2. Thus, the benefits and performance improvement due to the inclusion of a binding layer can be especially realized in the context of tissue sites or wounds that exude fluid having a high viscosity, such as some VLUs, which may often be due to a high protein concentration. Furthermore, the average pressure drop of 33.89 mmHg for Dressing 4 would likely be considered a test failure according to current testing standards for some therapy systems using certain negative-pressure sources. For example, testing standards for some therapy systems permit a maximum average pressure drop across dressing and/or system components of no more than 25 mmHg. Therefore, the inclusion of a binding layer of bacterial-binding material, such as that of Dressing 1 and Dressing 2, may offer considerable benefits for use with mechanically-driven sources of negative-pressure which may have lower tolerances for pressure drops.

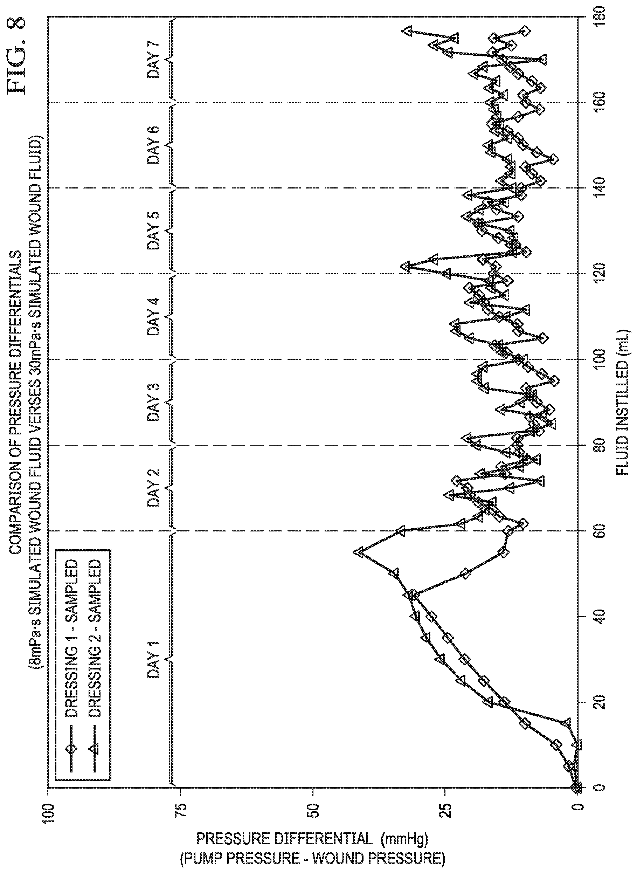

Figure 8 includes a chart, similar to those of Figures 6 and 7, comparing the results of Dressing 1, which was subjected to the 8 mPa·s simulated wound fluid, and Dressing 2, which was subjected to the 30 mPa·s simulated wound fluid. Given that each of Dressing 1 and Dressing 2 had the same structure and included one binding layer of Cutimed^{®} Sorbact^{®} DACC material sandwiched between three layers of manifold material, which were three layers of Libeltex TDL2 80gsm material, the effects of varied simulated wound fluid viscosity can be observed. The two dressings, Dressing 1 and Dressing 2, were tested according to the same testing protocols as the experiments of Figures 6 and 7. As shown in the chart of Figure 8, the average pressure drop for Dressing 1, which was subjected to the 8 mPa·s simulated wound fluid, was 12.94 mmHg as compared to the average pressure drop of 17.39 mmHg for Dressing 2, which was subjected to the 30 mPa·s simulated wound fluid.

Descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations one or more of the negative-pressure source 102, the dressing 104, and the container 106 may be separated from other components for manufacture or sale. In other example configurations, the controller 108 may also be manufactured, configured, assembled, or sold independently of other components.

The appended claims set forth novel and inventive aspects of the subject matter described above, but the claims may also encompass additional subject matter not specifically recited in detail.

## Claims

1. An apparatus for treating a tissue site, comprising:
a dressing (104, 504); and
a bridge (122, 222) adapted to be fluidly coupled to the dressing (104, 504), the bridge (122, 222) comprising:
a first manifold layer (350),
a second manifold layer (352),
a binding layer (370) comprising a binding material positioned between the first manifold layer (350) and the second manifold layer (352), wherein the binding material has bacterial-binding and protein-binding properties and
a sealing material having a first end (224) and a second end (226), wherein the first end (224) comprises a first aperture (330) adapted to be fluidly coupled to a negative-pressure source, and the second end (226) comprises a second aperture (332) adapted to be fluidly coupled to the dressing (104, 504);
wherein the first manifold layer (350), the second manifold layer (352), and the binding layer (370) are enclosed within the sealing material and are fluidly coupled between the first and second apertures (330, 332).

2. The apparatus of claim 1, further comprising an interface (336) adapted to be fluidly coupled to the first end (224) of the bridge (122, 222).

3. The apparatus of claim 1, wherein the binding material comprises dialkyl carbamoyl chloride.

4. The apparatus of claim 1, wherein the dressing further comprises:
a first layer comprising a tissue interface (114, 514);
a second layer comprising a manifold material (522) having a first surface adjacent the first layer and a second surface; and
a third layer comprising a polymer drape (516) positioned adjacent to the second surface of the second layer.

5. The apparatus of claim 4, wherein the tissue interface (114, 514) comprises a perforated silicone.

6. The apparatus of claim 4, wherein the tissue interface comprises a polyethylene film.

7. The apparatus of claim 4, wherein the tissue interface (114, 514) comprises an adhesive.

8. The apparatus of claim 1, wherein the first manifold layer (350) comprises a non-woven material.

9. The apparatus of claim 1, wherein the first manifold layer (350) comprises an open-celled polyurethane foam.

10. The apparatus of claim 1, wherein the second manifold layer (352) comprises a non-woven material.

11. The apparatus of claim 1, wherein the sealing material comprises a polyurethane film.

12. The apparatus of claim 1, wherein the binding material comprises fibers coated with dialkyl carbamoyl chloride.

13. The apparatus of claim 1, wherein the binding material comprises fibers coated with a hydrophobic coating.

14. The apparatus of claim 1, wherein the bridge further comprises a super-absorbent layer positioned between the first manifold layer (350) and the second manifold layer (352).

## Patentansprüche

1. Eine Einrichtung zum Behandeln einer Gewebestelle, aufweisend:
einen Verband (104, 504); und
eine Brücke (122, 222), die angepasst ist, um mit dem Verband (104, 504) fluidisch gekoppelt zu werden, die Brücke (122, 222) aufweisend:
eine erste Verteilerschicht (350),
eine zweite Verteilerschicht (352),
eine Bindeschicht (370), aufweisend ein Bindematerial, positioniert zwischen der ersten Verteilerschicht (350) und der zweiten Verteilerschicht (352), wobei das Bindematerial über bakterienbindende und proteinbindende Eigenschaften verfügt und
ein Dichtungsmaterial, das über ein erstes Ende (224) und ein zweites Ende (226) verfügt, wobei das erste Ende (224) eine erste Öffnung (330), die angepasst ist, um mit einer Unterdruckquelle fluidisch gekoppelt zu werden, aufweist, und das zweite Ende (226) eine zweite Öffnung (332), die angepasst ist, um mit dem Verband (104, 504) fluidisch gekoppelt zu werden, aufweist;
wobei die erste Verteilerschicht (350), die zweite Verteilerschicht (352) und die Bindeschicht (370) innerhalb des Dichtungsmaterials eingeschlossen sind und zwischen der ersten und der zweiten Öffnung (330, 332) fluidisch gekoppelt sind.

2. Die Einrichtung nach Anspruch 1, ferner aufweisend eine Schnittstelle (336), die angepasst ist, um mit dem ersten Ende (224) der Brücke (122, 222) fluidisch gekoppelt zu werden.

3. Die Einrichtung nach Anspruch 1, wobei das Bindematerial Dialkylcarbamoylchlorid aufweist.

4. Die Einrichtung nach Anspruch 1, wobei der Verband ferner aufweist:
eine erste Schicht, aufweisend eine Gewebeschnittstelle (114, 514);
eine zweite Schicht, aufweisend ein Verteilermaterial (522), das über eine erste Oberfläche angrenzend an die erste Schicht und eine zweite Oberfläche verfügt, und
eine dritte Schicht, aufweisend ein Polymerabdeckmaterial (516), das angrenzend an die zweite Oberfläche der zweiten Schicht positioniert ist.

5. Die Einrichtung nach Anspruch 4, wobei die Gewebeschnittstelle (114, 514) ein perforiertes Silikon aufweist.

6. Die Einrichtung nach Anspruch 4, wobei die Gewebeschnittstelle eine Polyethylenfolie aufweist.

7. Die Einrichtung nach Anspruch 4, wobei die Gewebeschnittstelle (114, 514) einen Klebstoff aufweist.

8. Die Einrichtung nach Anspruch 1, wobei die erste Verteilerschicht (350) ein Vliesmaterial aufweist.

9. Die Einrichtung nach Anspruch 1, wobei die erste Verteilerschicht (350) einen offenzelligen Polyurethanschaum aufweist.

10. Die Einrichtung nach Anspruch 1, wobei die zweite Verteilerschicht (352) ein Vliesmaterial aufweist.

11. Die Einrichtung nach Anspruch 1, wobei das Dichtungsmaterial eine Polyurethanfolie aufweist.

12. Die Einrichtung nach Anspruch 1, wobei das Bindematerial Fasern, die mit Dialkylcarbamoylchlorid beschichtet sind, aufweist.

13. Die Einrichtung nach Anspruch 1, wobei das Bindematerial Fasern, die mit einer hydrophoben Beschichtung beschichtet sind, aufweist.

14. Die Einrichtung nach Anspruch 1, wobei die Brücke ferner eine superabsorbierende Schicht, die zwischen der ersten Verteilerschicht (350) und der zweiten Verteilerschicht (352) positioniert ist, aufweist.

## Revendications

1. Appareil de traitement d'un site tissulaire, comprenant :
un pansement (104, 504) ; et
un pont (122, 222) conçu pour être accouplé fluidiquement au pansement (104, 504), le pont (122, 222) comprenant :
une première couche de collecteur (350),
une seconde couche de collecteur (352),
une couche de liaison (370) comprenant un matériau de liaison placé entre la première couche de collecteur (350) et la seconde couche de collecteur (352), dans lequel le matériau de liaison a des propriétés de liaison aux bactéries et des propriétés de liaison aux protéines, et
un matériau d'étanchéité ayant une première extrémité (224) et une seconde extrémité (226), dans lequel la première extrémité (224) comprend une première ouverture (330) conçue pour être accouplée fluidiquement à une source de pression négative, et la seconde extrémité (226) comprenant une seconde ouverture (332) conçue pour être accouplée fluidiquement au pansement (104, 504) ;
dans lequel la première couche de collecteur (350), la seconde couche de collecteur (352) et la couche de liaison (370) sont enfermées dans le matériau d'étanchéité et sont accouplées fluidiquement entre les première et seconde ouvertures (330, 332).

2. Appareil selon la revendication 1, comprenant en outre une interface (336) conçue pour être accouplée fluidiquement à la première extrémité (224) du pont (122, 222).

3. Appareil selon la revendication 1, dans lequel le matériau de liaison comprend du chlorure de dialkyl carbamoyle.

4. Appareil selon la revendication 1, dans lequel le pansement comprend en outre :
une première couche comprenant une interface tissulaire (114, 514) ;
une deuxième couche comprenant un matériau de collecteur (522) ayant une première surface à proximité de la première couche et une seconde surface ; et
une troisième couche comprenant un drapé polymère (516) placé à proximité de la seconde surface de la deuxième couche.

5. Appareil selon la revendication 4, dans lequel l'interface tissulaire (114, 514) comprend un silicone perforé.

6. Appareil selon la revendication 4, dans lequel l'interface tissulaire comprend un film de polyéthylène.

7. Appareil selon la revendication 4, dans lequel l'interface tissulaire (114, 514) comprend un adhésif.

8. Appareil selon la revendication 1, dans lequel la première couche de collecteur (350) comprend un matériau non tissé.

9. Appareil selon la revendication 1, dans lequel la première couche de collecteur (350) comprend une mousse de polyuréthane à alvéoles ouverts.

10. Appareil selon la revendication 1, dans lequel la seconde couche de collecteur (352) comprend un matériau non tissé.

11. Appareil selon la revendication 1, dans lequel le matériau d'étanchéité comprend un film de polyuréthane.

12. Appareil selon la revendication 1, dans lequel le matériau de liaison comprend des fibres revêtues de chlorure de dialkyl carbamoyle.

13. Appareil selon la revendication 1, dans lequel le matériau de liaison comprend des fibres revêtues d'un revêtement hydrophobe.

14. Appareil selon la revendication 1, dans lequel le pont comprend en outre une couche super-absorbante placée entre la première couche de collecteur (350) et la seconde couche de collecteur (352).
